(19) Europäisches Patentamt / European Patent Office / Office européen des brevets

(11) **EP 2 951 243 B1**

(12) **EUROPÄISCHE PATENTSCHRIFT**


(45) Veröffentlichungstag und Bekanntmachung des Hinweises auf die Patenterteilung:
**08.03.2017 Patentblatt 2017/10**

(21) Anmeldenummer: **14702255.2**

(22) Anmeldetag: **31.01.2014**

(51) Int Cl.:
***C09D 5/00*** *(2006.01)* ***C09D 5/16*** *(2006.01)*
***C09D 175/04*** *(2006.01)*

(86) Internationale Anmeldenummer:
**PCT/EP2014/051876**

(87) Internationale Veröffentlichungsnummer:
**WO 2014/118312 (07.08.2014 Gazette 2014/32)**


(54) **VERNETZTER KUNSTSTOFFWERKSTOFF MIT INTRINSISCH ANTIMIKROBIELLER WIRKUNG AUF BASIS VON VINYLESTERN UND VINYLESTERURETHANEN**

CROSSLINKED PLASTIC MATERIAL WITH INTRINSICALLY ANTIMICROBIAL EFFECT BASED ON VINYL ESTERS AND VINYL ESTERURETHANES

MATIÈRE PREMIÈRE SYNTHÉTIQUE MISE EN RÉSEAU AVEC ACTION ANTIMICROBIENNE INTRINSÈQUE À BASE D’ÉTHERS DE VINYLE ET URÉTHANES D’ÉTHER DE VINYLE


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.01.2013 EP 13000470**

(43) Veröffentlichungstag der Anmeldung:
**09.12.2015 Patentblatt 2015/50**



(73) Patentinhaber: **Fachhochschule Münster**
**48565 Steinfurt (DE)**

(72) Erfinder:
- **LORENZ, Reinhard**
  **48565 Steinfurt (DE)**
- **FISCHER, Björn**
  **48369 Saerbeck (DE)**
- **KREYENSCHMIDT, Martin**
  **49393 Lohne (DE)**
- **KREYENSCHMIDT, Judith**
  **53359 Rheinbach (DE)**

(74) Vertreter: **Ter Meer Steinmeister & Partner**
**Patentanwälte mbB**
**Nymphenburger Straße 4**
**80335 München (DE)**


(56) Entgegenhaltungen:
**DE-A1- 4 432 985** **DE-A1- 10 242 561**
**DE-C1- 19 723 504**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 951 243 B1

Printed by Jouve, 75001 PARIS (FR)

## Beschreibung

Die vorliegende Erfindung betrifft eine radikalisch härtbare chemische Zusammensetzung in Form eines Harzes zur Herstellung von Werkstoffen mit intrinsisch antimikrobieller Wirkung sowie die Anwendungen dieser Werkstoffe sowie ein Verfahren zur Herstellung dieser Harze und Werkstoffe sowie die Verwendung aminofunktionalisierter Styrolderivate als Reaktivverdünner.

Bei zahlreichen Anwendungen von Kunststoffen und Werkstoffen ist der Aufwuchs von Algen, Pilzen oder Muscheln eine unerfreuliche Begleiterscheinung. Deutlich ernstere Probleme können allerdings durch die Bildung von bakteriellen Biofilmen und die Übertragung pathogener Keime entstehen. Der Bekämpfung und Vermeidung dieser unerwünschten Effekte kommt somit große medizinische, hygienische, lebensmitteltechnologische und praktische Bedeutung zu: Man behilft sich im Allgemeinen mit mechanisch-chemischer Reinigung, mit Additivierung des Kunststoffs oder Lackes durch Zugabe klassischer Biozide (z.B. Silber-, Kupfer- oder Zinkverbindungen oder organische Verbindungen wie Triclosan, 10,10'-Oxy-bis-phenoxyarsin, N-(Trifluormethylthio)phthalimid, N-(Trichlormethylthio)-phthalimid, verschiedene Isothiazolione) sowie mit fortgesetzter Verwendung verschiedener äußerer Desinfektionsmittel - wie es beispielsweise in Krankenhäusern oder auch in der Lebensmittel- und Fleischverarbeitung gängige Praxis ist.

Es war bislang schwierig, Oberflächen antimikrobiell auszustatten, ohne dass der als Additiv zugegebene antimikrobielle Wirkstoff aus der Oberfläche herausdiffundiert. Es gab einige Versuche, antimikrobiell funktionalisierte Monomere zum Aufbau von antimikrobiellen Polymeren zu nutzen. Eines dieser Monomere war beispielsweise tert.-Butylaminoethylmethacrylat (TBAEMA). Der Nachteil dieser bislang verfügbaren Polymere, z.B. Poly(TBAEMA), lag darin, dass die Glasübergangstemperatur sehr niedrig lag, wie beispielsweise unterhalb von 50 °C bei Poly(TBAEMA).

US 6,242,526 beschreibt antimikrobielle Polymerlatices, die sich aus einer ethylenisch ungesättigten Säure als Anion und einer quaternären Ammoniumverbindung als Kation zusammensetzen, wobei die antimikrobielle Wirkung der quaternären Ammoniumverbindungen zuzuordnen ist.

US 4,810,567 beschreibt antimikrobielle Textilien, wobei verschiedene Basistextilien durch eine graft-Copolymerisation mit Säuregruppen enthaltenden Monomeren funktionalisiert werden, an die durch Amidierung antimikrobielle Proteine und Antibiotika gebunden werden.

US 5,614,568 beschreibt einen antibakteriellen, thermoplastischen Compound, der unter anderem Silber-, Kupfer- oder Zinkzeolithe enthält. Zudem werden polymere und niedermolekulare Additive mit speziellen funktionellen Gruppen verwendet, die die antimikrobielle Wirkung steigern.

US 4,447,580 beschreibt ein Copolymer auf Acrylat-Basis enthaltend aminofunktionalisierte Styrol-Monomereinheiten zur Herstellung von Tauchlacken für die Kataphorese. Diese Copolymere werden über eine thermisch aktivierte MichaelAddition mit oligomeren und polymeren Verbindungen umgesetzt, die aktivierte Doppelbindungen enthalten. Die aminofunktionalisierten Styrolderivate werden nicht für eine radikalische Vernetzung genutzt und auch nicht für eine antimikrobielle Wirkung.

JP 2000 239 281 A beschreibt die Synthese von aminofunktionalisierten Styrolderivaten, aus denen vernetzte Polymere hergestellt werden, die in immobilisierte polymere Lithiumamide überführt werden. Diese dienen der organischen Synthese.

US 6,200,680 beschreibt die Herstellung von Zinkoxid-Partikeln unter Verwendung polymerer Hilfsstoffe, darunter aminofunktionalisierter Polymere auf der Basis von aminofunktionalisierten Styrolderivaten.

US 4,021,416 beschreibt aminoethanthiol-funktionalisierte Styrolderivate, die als Komplexbildner für Silberionen und/oder lösliche Silberkomplexe in der Fotografie genutzt werden können.

Kuno et al. (Reactive & Functional Polymers 43 (2000) 43 - 51) beschreiben Poly[N-(p-vinylbenzyliden)-tert.-butylaminoxid] als neuen Radikalfänger für Anwendungen in der Umwelttechnologie.

DE 102 42 561 A1 offenbart eine antimikrobielle Beschichtung, wobei die antimikrobielle Beschichtung Polymere von speziellen cyclischen Aminen mit zumindest einer polymerisierbaren ungesättigten Gruppe aufweist. Weiterhin wird ein Verfahren zur Herstellung dieser antimikrobiellen Beschichtungen und deren Verwendung zur Herstellung von Erzeugnissen mit antimikrobiellen Eigenschaften beschrieben.

DE 44 32 985 A1 offenbart Bindemittel und deren Herstellung sowie deren Verwendung in Überzugsmitteln für kratz- und säurefeste Überzüge. Die Bindemittel werden durch eine radikalische Polymerisation von a) (Meth)acrylmonomer und gegebenenfalls weiterem radikalisch polymerisierbaren Monomer(en) in Gegenwart von b) Cycloolefinhomopolymer und/oder Cycloolefincopolymer, die frei von olefinischen Doppelbindungen sind, gewonnen.

DE 197 23 504 C1 offenbart ein Beschichtungsmittel insbesondere zur Beschichtung von Kunststoffen, Verfahren zu seiner Herstellung und die Verwendung als Deck- oder Klarlack. Die Beschichtungsmittel enthalten

a) ein oder mehrere spezielle Polyersterharze,
b) ein oder mehrere spezielle Polyacrylatharze,
c) ein oder mehrere Di- und/oder Polyisocyante
d) ein oder mehrere Lichtschutzmittel auf Basis eines UV-Absorbers

e) ein oder mehrere spezielle Lichtschutzmittel auf Basis sterisch gehinderter Amine, und

f) ein oder mehrere organische Lösungsmittel.

H. Menzel gibt in "Schutzschicht gegen Bakterien" (Nachrichten aus der Chemie, 59, November 2011, S. 1039-43) einen Überblick über medizinisch geeignete antimikrobielle Beschichtungen auf der Basis von Polymeren mit hydrophoben Strukturelementen in Kombination mit einer hohen positiven Ladungsdichte. Die verschiedenen Wirkmechanismen gegenüber gramm-positiven und gramm-negativen Bakterien werden kurz erörtert. Die meisten Strukturen beruhen auf quaternären Ammonium- und Phosphoniumionen. Antimikrobielle Polymere für kompakte werkstoffliche Anwendungen werden nicht behandelt.

Die Aufgabe der vorliegenden Erfindung besteht darin, eine in der Praxis leicht einsetzbare Technologie bereitzustellen, mit der antimikrobiell ausgerüstete Oberflächen und Erzeugnisse hergestellt werden können, bei denen kein antimikrobielles Additiv aus der Oberfläche oder den Erzeugnissen herausdiffundieren kann. Eine weitere Aufgabe besteht darin, einen antimikrobiellen Werkstoff bereitzustellen, der "in the bulk", d.h. im Gesamtvolumen antimikrobiell funktionalisiert ist und der daher nach Verletzung oder Veränderung seiner Oberfläche, z.B. durch Schlag, Abrasion oder Schnitt, eine neue Oberfläche bildet, die ebenfalls antimikrobiell ist.

Die der Erfindung zugrunde liegende Aufgabe wird in einer ersten Ausführungsform gelöst durch eine Harz-Zusammensetzung zur Herstellung von Erzeugnissen mit antimikrobieller Wirkung enthaltend

a) Vinylester (VE) und/oder Vinylesterurethan (VEU), und

b) Styrolderivat als Reaktivverdünner, wobei bevorzugt je Doppelbindung in Komponente a) 0,5 bis 8 Styrolderivatmoleküle in der Zusammensetzung vorhanden sind,

wobei die Komponente b) aminofunktionalisiert ist und die Aminofunktionaliät unter die Formel

$$-(CH_2)_q-NH_pR^1R^2A_p$$

fällt, wobei

q entweder 1 oder 2 ist,

p 0 oder 1 ist,

$R^1$ ausgewählt ist aus H, linearen oder verzweigten oder zyklischen Alkylresten aufweisend 1 bis 10 Kohlenstoffatome,

$R^2$ ein linearer oder verzweigter oder zyklischer Alkylrest aufweisend 1 bis 10 Kohlenstoffatome ist,

A das Anion einer Säure ist und,

der Amin-Stickstoff N der obenstehenden Formel neutral (p = 0) oder positiv (p = 1) geladen ist.

Die nach der Härtung dieser erfindungsgemäßen VE- bzw. VEU-Harz-Zusammensetzungen gebildeten, vernetzten Kunststoffwerkstoffe wirken ohne Verwendung zusätzlicher Biozide intrinsisch antimikrobiell. Die neuen Werkstoffe werden daher als intrinsisch antimikrobiell bezeichnet. Der Vorteil der erfindungsgemäßen VE- bzw. VEU-Harz-Zusammensetzung ist, dass hiermit Erzeugnisse hergestellt werden können, aus denen antimikrobielle Wirkstoffe nicht austreten können und dass diese Erzeugnisse auch bei mechanischer Veränderung oder Beschädigung ihrer Oberfläche antimikrobiell bleiben.

Die Zusammensetzung im Sinne der Erfindung enthält einen Vinylester oder Vinylesterurethan und wenigstens einen Reaktivverdünner.

Es zeigte sich, dass die der Erfindung zugrunde liegende Aufgabe mit radikalisch vernetzenden Harzen vom Typ der Vinylesterharze (VE-Harze) und Vinylesterurethanharze (VEU-Harze) gelöst werden kann. Dazu wird der üblicherweise eingesetzte Reaktivverdünner Styrol durch ein aminofunktionalisiertes Styrolderivat, eine Mischung verschiedener aminofunktionalisierter Styrolderivate, eine Mischung eines oder mehrerer aminofunktionalisierter Styrolderivate mit einem oder mehreren aminofunktionalisierten Methacrylaten oder eine dieser Mischungen mit weiteren Reaktivverdünnern ersetzt. Zu den weiteren Reaktivverdünnern zählen beispielweise Styrol, Methylstyrol, Vinyltoluol, tert.-Butylstyrol, 4-Vinylpyridin, 3-Vinylpyridin, 2-Vinylpyridin, Methylmethacrylat, Divinylbenzol, 1,2,4-Trivinylcyclohexan, Diallylphthalat, Diallylisophthalat, Trisallylisocyanurat.

Zusätzlich kann das Vinylesterurethan im VEU-Harz Aminogruppen enthalten. Weiterhin können die erfindungsgemäßen antimikrobiell ausgestatteten VE-Harze und VEU-Harze miteinander gemischt und nachfolgend gehärtet werden. Erfindungsgemäß sind ebenfalls Mischungen dieser intrinsisch antimikrobiell ausgerüsteten Harze mit intrinsisch antimikrobiellen UP-Harzen möglich.

Darüber hinaus sind Mischungen mit thermoplastischen Polymeren und intrinsisch antimikrobiellen thermoplastischen Polymeren erfindungsgemäß, beispielsweise zur Zähmodifizierung oder zur Schwundkompensation der gebildeten Duromere. Auch sind Mischungen dieser antimikrobiell ausgerüsteten VE- und VEU-Harze mit konventionellen, nicht antimikrobiell ausgerüsteten Harzen vom Typ der UP-Harze, VE-Harze, VEU-Harze und Methacrylatharze erfindungsgemäß möglich, soweit der antimikrobielle Effekt erhalten bleibt.

**Vinylesterharz (VE-Harz) und Vinlyesterurethanharz (VEU-Harz)**

Das VE-Harz im Sinne der Erfindung besteht beispielsweise aus dem Vinylester und dem Reaktivverdünner. Bei den Vinylestern handelt es sich beispielsweise um Reaktionsprodukte von Bisphenol A-, Bisphenol F- und Novolak-basierten Glycidylethern mit Methacrylsäure, wobei sich der Methacrylsäureester bildet. Auch können Glycidylether anderer Bisphenole (z.B. Bisphenol TMC) eingesetzt werden. Nach der radikalischen Härtung (chemische Vernetzung) spricht man vom VE-Duromer oder VE-Netzwerk. Die Bisphenol A-basierten VE-Harze werden auch als VE/BA Harze bezeichnet; die Novolak-basierten Harze werden beispielsweise als VE/NO Harze bezeichnet (AVK-TV-Handbuch, "Faserverstärkte Kunststoffe und duroplastische Formassen", 2. Auflage 2005, Seite 40).

Das VEU-Harz im Sinne der Erfindung besteht beispielsweise aus dem Vinylesterurethan und dem Reaktivverdünner. Bei den Vinylesterurethanen handelt es sich beispielsweise entweder um Umsetzungsprodukte aus Hydroxypropylmethacrylat und/oder Hydroxyethylmethacrylat mit Diisocyanaten und/oder Triisocyanaten und/oder Polyisocyanaten. Alternativ kommen Umsetzungsprodukte aus Diolen und/oder Triolen und/oder Tetrolen unterschiedlicher Kettenlänge, Hydroxypropylmethacrylat und/oder Hydroxyethylmethacrylat sowie Diisocyanaten oder Triisocyanaten zum Einsatz. Eine geeignete Auswahl der Diole, Triole, Tetrole und Isocyanate erlaubt es beispielsweise, die Netzwerkeigenschaften in weiten Grenzen maßgeschneidert auf die Erfordernisse verschiedenster Anwendungen einzustellen. Nach der radikalischen Härtung (chemische Vernetzung) spricht man vom VEU-Duromer oder VEU-Netzwerk. Die bei Vinylesterurethanen eingesetzten Diole, Triole und Tetrole werden auch als Kettenverlängerer bezeichnet, und zwar unabhängig von ihrer Molmasse.

Vorzugsweise kann das Vinylesterurethan in Komponente a) Aminogruppen enthalten. Besonders bevorzugt können die Aminogruppen durch Verwendung von Kettenverlängeren wie beispielsweise Bishydroxyethylmethylamin oder Bishydroxyethyl-tert.-butylamin eingeführt werden. Die Kettenverlängerer können auch aus verschiedenen Diolen, Triolen und/oder Tetraolen zusammengesetzt sein.

VE-Harze und VEU-Harze haben wesentliche strukturelle Gemeinsamkeiten, weshalb sie erfindungsgemäß zusammengehören: VE- und VEU-Harze tragen die radikalisch reaktiven Doppelbindungen beispielsweise in Form einer Methacrylat-Funktion jeweils am Kettenende des oligomeren Epoxids bzw. Urethans. Dabei kann es sich um lineare Epoxide und Urethane handeln mit jeweils zwei Kettenenden, um trifunktionelle mit jeweils drei Kettenenden, um tetrafunktionelle mit vier Kettenenden, und so fort. Auch Mischungen können eingesetzt werden.

Radikalisch reaktive Doppelbindungen in der Molekülkette - wie sie ungesättigte Polyesterharze (UP-Harze) zeigen - sind bei VE- und VEU-Harzen in der Regel nicht vorhanden. Dies hat verschiedene Vorteile: Nach der Vernetzung zeigen VE-Duromere und VEU-Duromere relativ wenig freie Kettenenden im Netzwerk verglichen mit den Duromeren der UP-Harze. Dies erhöht die chemische Beständigkeit und die Zähigkeit des Netzwerks im Vergleich zu Netzwerken der UP-Harze. Auch ist der Glasübergang der VE- und VEU-Harze enger und schärfer. Daher werden in der Harzerzeugenden und in der Harz-verarbeitenden Industrie beide Stoffklassen als Einheit gesehen. In der Literatur wird beispielsweise ausgeführt: "Vinylesterharze, die mit Diisocyanat-Abkömmlingen aufgebaut sind, bilden als Vinylester-Urethan-Harze (VEU-Harze) eine besondere Gruppe, deren Formstoffe ähnliche Eigenschaften aufweisen wie die VE/BA-Harze und daher zum gleichen Harztyp gehören." (AVK-TV-Handbuch, "Faserverstärkte Kunststoffe und duroplastische Formassen" Auflage 2005, Seite 40).

Der Glycidylether im Sinne der Erfindung kann auch eine Mischung verschiedener Glycidylether sein.

Vorzugsweise handelt es sich bei dem Vinylester in Komponente a) um einen Vinylester aus einerseits di-, tri- und/oder höherfunktionellem Glycidylether eines di-, tri- und/oder höherwertigen Phenols und andererseits Methacrylsäure im molaren Verhältnis 1,25:1 bis 0,75:1.

Bei den Vinylesterurethanen in Komponente a) handelt es sich vorzugsweise entweder um Umsetzungsprodukte aus Hydroxypropylmethacrylat und/oder Hydroxyethylmeth-acrylat mit Diisocyanaten und/oder Triisocyanaten und/oder Polyisocyanaten. Alternativ kommen beispielsweise Umsetzungsprodukte aus Diolen und/oder Triolen und/oder Tetrolen unterschiedlicher Kettenlänge, Hydroxypropylmethacrylat und/oder Hydroxyethylmethacrylat sowie Diisocyanaten oder Triisocyanaten zum Einsatz.

Als Diisocyanate dienen vorzugsweise Monomer-MDI, Polymer-MDI (MDI: Methylen-diphenyldiisocyanat), 2,4-TDI, 2,6-TDI (TDI: Toluylendiisocyanat), IPDI, Oligomerisierungsprodukte des IPDI (IPDI: Isophorondiisocyanat), HDI, Oligomerisierungsprodukte des HDI (HDI: Hexamethylendiisocyanat), wobei es sich bei den Oligomerisierungsprodukten des IPDI und des HDI beispielsweise um die entsprechenden Isocyanurate und Biurete handelt, die in verschiedenen Viskositäten und Funktionalitäten kommerziell verfügbar sind. Zudem sind auch weitere Di- und Triisocyanate, die nicht zur Gruppe MDI, TDI, IPDI oder HDI gehören, erfindungsgemäß geeignet.

Die Kettenverlängerer sind vorzugsweise Propylenglykol, Dipropylenglykol, Tripropylenglykol, oligomere Propylenglykole, polymere Propylenglykole, Ethylenglykol, Diethylenglykol, Triethylenglykol, oligomere Polyethylenoxide, polymere Polyethylenoxide, Neopentylglykol, Hydroxypivalinsäureneopentylglykolester, 1,4-Butandiol, 1,3-Butandiol, 1,2-Butandiol, 2,3-Butandiol, 1,5-Petandiol, 1,6-Hexandiol, Cyclohexandimethanol, Tricyclodecandimethanol, Isosorbid, Bishydroxyethylanilin, Bishydroxypropylanilin oder beliebige Mischungen dieser Stoffe.

Zur Herstellung der Vinylesterurethane wird vorzugsweise ein Lösungsmittel, mehr bevorzugt ein aprotisches Lösungsmittel, verwendet, bei dem es sich beispielsweise um Aceton oder Methylethylketon handeln kann. Alternativ kann auch ein Reaktivverdünner eingesetzt werden, vorausgesetzt dieser reagiert nicht mit dem Isocyanat. Daher darf dieser Reaktivverdünner vorzugsweise keine N-H-Funktion aufweisen. Somit ist beispielsweise Dimethylaminomethylstyrol als Lösungsmittel geeignet, tert.-Butyl-aminomethylstyrol aber nicht. Man stellt beispielsweise eine flüssige homogene Mischung aus Hydroxypropylmethacrylat und/oder Hydroxyethylmethacrylat, dem oder den Kettenverlängerern, einem Urethanisierungskatalysator und dem Lösungsmittel her, in die man das flüssige Isocyanat (ggf. bei erhöhter Temperatur) so zutropft, dass eine Temperatur von etwa 60-80 °C nicht überschritten wird. Anschließend wird beispielsweise noch etwa 1 h bei 60-80 °C nachgerührt, um die Reaktion abzuschließen. Danach wird der flüssige Reaktivverdünner, z.B. tert.-Butylaminomethylstyrol, zugegeben und das VEU-Harz auf Raumtemperatur abgekühlt. Falls mit einem niedrig siedenden Lösungsmittel wie Aceton oder Methylethylketon gearbeitet wurde, wird dies beispielsweise im Vakuum abdestilliert.

**Reaktivverdünner**

Als Reaktivverdünner wird im Sinne der Erfindung in diesem Zusammenhang sowohl ein einzelnes geeignetes Monomer als auch eine geeignete Monomermischung bezeichnet. Entscheidend ist, dass das Monomer oder die Monomermischung eine radikalische Vernetzung erlauben und vollständig oder weitgehend vollständig ins Netzwerk eingebaut werden.

Die Zusammensetzung kann neben dem Styrolderivat b) einen weiteren oder weitere Reaktivverdünner aus der Gruppe der Styrolderivate und/oder Methacrylate und/oder höherfunktionelle Monomere beinhalten.

Das Verhältnis von Doppelbindung in Komponente a) zu Styrolderivatmolekül ist grundsätzlich zunächst nicht beschränkt. In einer bevorzugten Ausführungsform sind je Doppelbindung in Komponente a) 0,5 bis 8, bevorzugt 0,7 bis 7, mehr bevorzugt 1 bis 6, noch mehr bevorzugt 1,5 bis 4, insbesondere 2,0 bis 3,5 Styrolderivatmoleküle in der Zusammensetzung vorhanden. Liegt die Zahl darunter, so kann gegebenenfalls die Zusammensetzung zu hochviskos werden oder es kann zu ungenügender Vernetzung kommen. Liegt die Zahl darüber, so kann es sein, dass die Zusammensetzung gegebenenfalls zu niederviskos wird oder dass Styrolderivatmoleküle nicht vollständig bei der Vernetzungsreaktion reagieren und freies Restmonomer aus dem Erzeugnis oder der Oberfläche herausdiffundieren kann. Um dies zu verhindern, sollte der Restmonomergehalt vorzugsweise durch eine längere Nachhärtung bei erhöhter Temperatur abgesenkt werden.

**Aminofunktionalität**

Die der Erfindung zu Grunde liegende Aufgabe wird in einer zweiten Ausführungsform gelöst durch VE-Harze bzw. VEU-Harze zur Herstellung von Erzeugnissen mit antimikrobieller Wirkung enthaltend

a) einen Vinylester und/oder Vinylesterurethan und

b) Styrolderivat, wobei je Doppelbindung in Komponente a) 0,5 bis 8 Styrolderivatmoleküle in der Zusammensetzung vorhanden sind,

wobei das Styrolderivat aminofunktionalisiert ist,
wobei die Aminofunktionaliät unter die Formel

$$-(CH_2)_q-NH_pR^1R^2A_p$$

fällt, wobei

q entweder 1 oder 2 ist,

p 0 oder 1 ist,

$R^1$ ausgewählt ist aus H, linearen oder verzweigten oder zyklischen Alkylresten aufweisend 1 bis 10 Kohlenstoffatome,

$R^2$ ein linearer oder verzweigter oder zyklischer Alkylrest aufweisend 1 bis 10 Kohlenstoffatome ist,

A das Anion einer Säure ist und,

der Amin-Stickstoff N der obenstehenden Formel neutral (p = 0) oder positiv (p = 1) geladen ist.

p kann bevorzugt nach der Neutralisation mit einer Säure HA gleich 1 sein.

$R^2$ der Aminofunktionalität weist vorzugsweise 1 bis 10 Kohlenstoffatome auf und ist besonders bevorzugt verzweigt, ganz besonders bevorzugt ausgewählt aus iso-Propyl, tert.-Butyl oder tert.-Pentyl.

$R^1$ ist vorzugsweise ausgewählt aus H oder $R^2$.

Sofern $R^2$ mehr als drei Kohlenstoffatome aufweist, dann ist $R^1$ vorzugsweise ausgewählt aus H oder einem Alkylrest mit 1 bis 3 Kohlenstoffatomen.

Im Fall, dass Komponente a) ein Vinylester ist, kann es sich bei der Säure um Kohlensäure handeln, die in Gegenwart von Umgebungsluft oder Wasser in situ gebildet wird. In diesem Fall liegt eine Mischung aus neutralisiertem und nicht neutralisiertem Amin vor. Bei dem an der Umgebungsluft oder in Gegenwart von Wasser neutralisierten Amin ist das Gegenion A beispielsweise vor allem $HCO_3^-$.Eine weitere Neutralisation ergibt sich beispielsweise durch Reaktion mit Methacrylsäure, die nicht mit dem Glycidylether abreagiert hat. In diesem Fall ist das Gegenion $CH_2=C(CH_3)-COO^-$. Auch die Anwendung anderer Säuren ist möglich.

Im Fall, dass Komponente a) ein Vinylesterurethan ist, kann es sich bei der Säure beispielweise um Kohlensäure handeln, die in Gegenwart von Umgebungsluft oder Wasser in situ gebildet wird. In diesem Fall liegt eine Mischung aus neutralisiertem und nicht neutralisiertem Amin vor. Bei dem an der Umgebungsluft oder in Gegenwart von Wasser neutralisierten Amin ist das Gegenion A vor allem $HCO_3^-$. Auch die Zugabe und Anwendung anderer mono- oder höherfunktioneller Säuren ist möglich.

Da die Styrolmonomere den Vinylester bzw. das Vinylesterurethan in der Regel in einem breiten Zusammensetzungsbereich problemlos quer vernetzten, kann man über den Gehalt an aminofunktionalisiertem Reaktivverdünner besonders gut den Gehalt an Aminofunktionen im entstehenden vernetzten Kunststoff steuern.

**Vinylesterurethan in Komponente a)**

Das Vinylesterurethan kann erfindungsgemäß auch Aminogruppen enthalten. Dazu eignen sich beispielsweise aminohaltige Diole und Triole sowie Aminoalkohole mit einer NH-Struktur und einem zusätzlichen tertiären Amin. Beispiele für diese Kettenverlängerer sind: N-Methyldiethanolamin, N-tert.-Butyldiethanolamin, N-Methyldiiso-propanolamin, N-tert.-Butyldiisopropanolamin, 3-(Diethylamino)-1,2-propandiol, N-Hydroxyethylpiperazin, N,N'-Bishydroxyethylpiperazin, N-Hydroxy-propylpiperazin, N,N'-Bishydroxypropylpiperazin, N,N-Dimethyl-N',N'-bishydroxyethyl-1,3-diamino-propan, N,N-Dimethyl-N',N'-bishydroxyethyl-diaminoethan, Triethanolamin, Triisopropanolamin.

Eine weitere Möglichkeit besteht in der Verwendung aminogruppenhaltiger Polyesterole als Kettenverlängerer, die zum Beispiel durch folgende Reaktion zugänglich sind: Maleinsäureanhydrid, Maleinsäure oder Fumarsäure werden mit einem stöchiometrischen Überschuss eines Diols, z.B. Ethylenglykol, Diethylenglykol, Propylenglykol, Dipropylenglykol oder Neopentylglykol zu einem Polyester mit einer Säurezahl < 1 mg KOH/g durch Schmelzkondensation umgesetzt. Die Doppelbindungen werden in einer nachfolgenden Michaeladdition quantitativ oder weitgehend quantitativ mit einem Dialkylamin, z.B. Dimethylamin, Diethylamin, Diisopropylamin, umgesetzt. Für diese Reaktion wird das Dialkylamin stöchiometrisch im Überschuss eingesetzt; nach Beendigung der Reaktion wird der Überschuss an Dialkylamin im Vakuum abgezogen. Das entstandene aminogruppenhaltige Polyesterol ist mit Hydroxylgruppen terminiert und kann nach Bestimmung der OH-Zahl als Kettenverlängerer eingesetzt werden.

**Aminofunktionalisiertes Styrolderivat**

In der erfindungsgemäßen Zusammensetzung ist vorzugsweise das Styrolderivat aminofunktionalisiert. Das Molekulargewicht des aminofunktionalisierten Styrolderivats liegt vorzugsweise in einem Bereich von 100 bis 300 g/mol, insbesondere in einem Bereich von 170 bis 250 g/mol. Es hat sich herausgestellt, dass die Vernetzungsreaktion bei einem zu hohen Molekulargewicht des Monomers unvollständig ablaufen kann.

Das aminofunktionalisierte Styrolderivat weist vorzugsweise 10 bis 20 Kohlenstoffatome, insbesondere 12 bis 18 Kohlenstoffatome auf.

Das erfindungsgemäße aminofunktionalisierte Styrolderivat ist vorzugsweise ausgewählt aus der Gruppe N-(4-ethenylbenzyl)-2-methylpropan-2-amin (auch tert.-Butyl-Aminomethyl-Styrol oder TBAMS), N-(4-ethenylbenzyl)ethanamin (auch Ethyl-Aminomethyl-Styrol oder EAMS),N-(4-ethenylbenzyl)propan-1-amin (auch n-Propyl-Aminomethyl-Styrol oder PAMS), N-(4-ethenylbenzyl)propan-2-amin (auch iso-Propyl-Aminomethyl-Styrol oder IPAMS), N-(4-ethenylbenzyl)butan-1-amin (auch n-Butyl-Aminomethyl-Styrol oder BAMS), N-(4-ethenylbenzyl)butan-2-amin (auch sec-Butyl-Aminomethyl-Styrol oder SBAMS), N-(4-ethenylbenzyl)-2-methylpropan-1-amin (auch iso-Butyl-Aminomethyl-Styrol oder IBAMS), N-(4-ethenylbenzyl)pentan-1-amin (auch n-Pentyl-Aminomethyl-Styrol oder PENAMS), N-(4-ethenylbenzyl)-3-methylbutan-1-amin (auch iso-Pentyl-Aminomethyl-Styrol oder IPENAMS), N-(4-ethenylbenzyl)pentan-3-amin (auch 3-Pentyl-Aminomethyl-Styrol oder 3-PENAMS), N-(4-ethenylbenzyl)-2-methylbutan-2-amin (auch tert.-Pentyl-Aminomethyl-Styrol oder TPAMS), N-(4-ethenyl-benzyl)cyclopentanamin (auch Cyclopentyl-Aminomethyl-Styrol oder CPENAMS), N-(4-ethenylbenzyl)cyclohexanamin (auch Cyclohexyl-Aminomethyl-Styrol oder CHAMS), N-(4-ethenylbenzyl)-N,N-dimethylamin (auch Dimethyl-Aminomethyl-Styrol oder DMAMS, N-(4-ethenylbenzyl)-N,N-diethylamin (auch Diethyl-Aminomethyl-Styrol oder DEAMS), N-(4-ethenylbenzyl)-N-(propan-2-yl)propan-2-amin (auch Diisopropyl-Aminomethyl-Styrol oder DIPAMS) und Mischungen daraus, wobei diese Verbindungen wie folgt abgebildet sind:

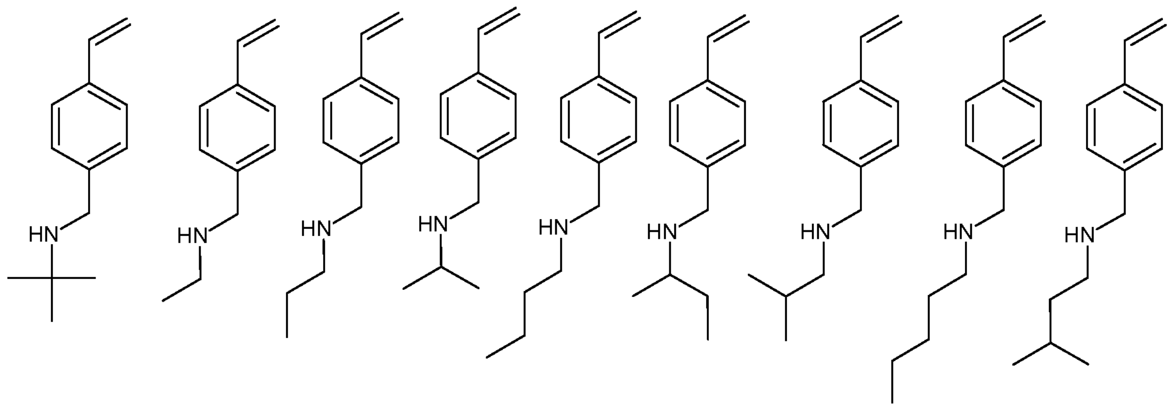

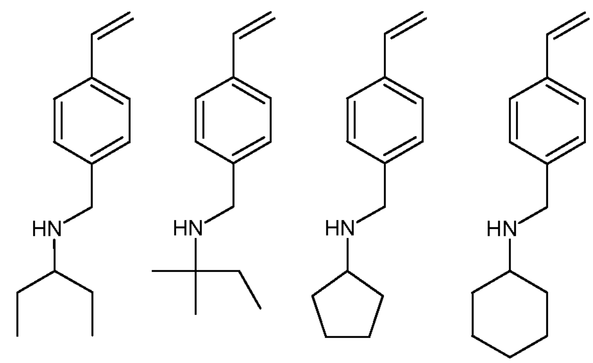

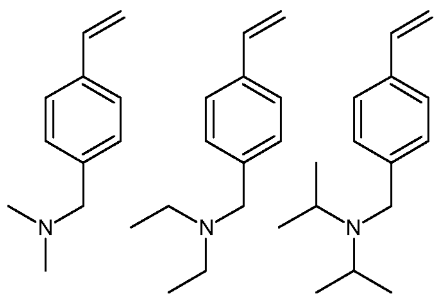

Neben den bereits genannten para-Isomeren können erfindungsgemäß auch alle meta-Isomere sowie alle ortho-Isomere der bereits genannten Derivate sowie beliebige Mischungen aller ortho-, meta- und para-Isomere verwendet werden.

In einer Ausführungsform wird die der Erfindung zu Grunde liegende Aufgabe auch gelöst durch Verwendung eines oder einer Mischung dieser drei Stoffe

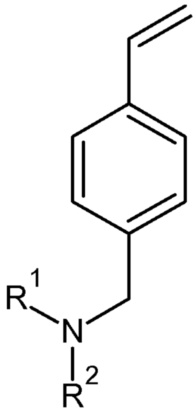

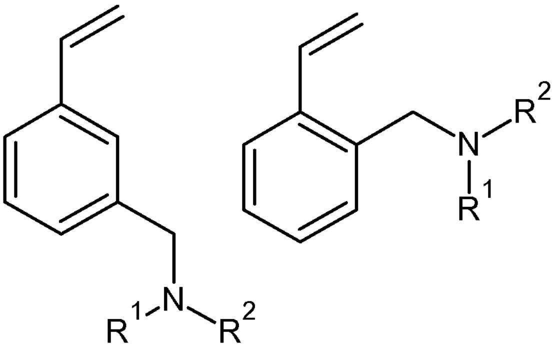

als Reaktivverdünner,
wobei $R^1$ und/oder $R^2$ unabhängig ausgewählt sind aus der Gruppe Methyl, Ethyl, n-Propyl, iso-Propyl, n-Butyl, sec-Butyl, iso-Butyl, tert.-Butyl, n-Pentyl, 3-Pentyl, iso-Pentyl, tert.-Pentyl, Cyclopentyl, Cyclohexyl,
wobei $R^1$ auch H sein kann.

Diese Monomere haben sich bei der Entwicklung der erfindungsgemäßen Werkstoffe als besonders geeignet erwiesen, antimikrobielle Werkstoffe zu erzeugen.

Ganz besonders bevorzugt ist die Verwendung der Stoffe mit $R^1$ und/oder $R^2$ ausgewählt aus Ethyl, iso-Propyl, tert.-Butyl, tert.-Pentyl,
wobei $R^1$ auch H sein kann.

In einer ganz besonders bevorzugten Ausführungsform ist $R^1$ gleich Wasserstoff und $R^2$ ist ausgewählt aus iso-Propyl, tert.-Butyl, tert.-Pentyl.

In der vorliegenden Harz-Zusammensetzung sind bevorzugt je Doppelbindung in Komponente a) 0,5 bis 8 Styrolderivatmoleküle (Komponente b)) vorhanden. Anders gesagt ist somit das Verhältnis von Styrolderivatmolekülen b) zu Doppelbindung aus Komponente a) 0,5:1 bis 8:1. Die Anzahl der Styrolderivatmoleküle/Doppelbindungen in Komponente a) sind proportional zu den entsprechenden Molmengen, so dass sich das genannte Verhältnis auch mit deren Hilfe berechnen lässt.

Die Molmenge des Styrolderivats (Komponente b)) lässt sich aus der eingesetzten Menge und ihrer Molmasse gemäß

$$n_{Sty}=m_{Sty}/M_{Sty}$$

bestimmen, wobei $m_{Sty}$ die eingesetzte Menge und $M_{Sty}$ das Molgewicht des Styrolderivats sind.

Die Molmenge der Doppelbindungen in Komponente a) lässt sich aus der eingesetzten Menge und der Molmasse der Komponente a) gemäß

$$n_{Dop}=f\cdot m_{Dop}/M_{Dop}$$

bestimmen, wobei f die Anzahl Doppelbindungen in einem Molekül der Komponente a) sowie $m_{Dop}$ die eingesetzte Menge und $M_{Dop}$ das Molgewicht der Komponente a) sind.

**Zusammensetzung**

Die Zusammensetzung enthält vorzugsweise wenigstens 20 Gew.%, besonders bevorzugt wenigstens 50 Gew.%, ganz besonders bevorzugt wenigstens 80 Gew.% einer Mischung der Komponenten a) und b). Im Übrigen kann die Zusammensetzung beispielsweise andere Monomere, Oligomere, Polymere, Lichtschutzmittel, Initiatoren, Additive, Pigmente, Trennmittel, Rheologieadditive, Fasern und/oder Füllstoffe enthalten.

Auf 100 Gewichtsteile einer Mischung aus den Komponenten a) und b) kommen in der Zusammensetzung

vorzugsweise 0,2 bis 4 Gewichtsteile Radikalinitiator. Dieser Radikalinitiator ist vorzugsweise kein peroxidischer Radikalinitiator. Besonders bevorzugt ist der Radikalinitiator ein Photoinitiator, z.B. ein Derivat des Benzoins, des Benzils oder ein α-Hydroxyketon oder ein α-Aminoketon oder ein Acylphosphinoxid oder ein Bisacylphosphinoxid. Dem Fachmann ist eine Vielzahl von Photoinitiatoren bekannt. Photoinitiatoren, die C-Radikale bilden, sind bevorzugt. Besonders bevorzugt sind C-Radikalgeneratoren vom Typ der Azoinitiatoren, z.B. 2,2'-Azobis(2-methyl-propionitril), auch als AIBN bezeichnet, 1,1'-Azobis(cyclohexan-1-carbonitril) oder Dimethyl-2,2'-azobis(2-methylpropionat) und sogenannte C-C-labile Verbindungen, z.B. 2,3-Dimethyl-2,3-diphenylbutan oder 3,4-Dimethyl-3,4-diphenylhexan.

Beim Radikalinitiator kann es sich auch um eine Mischung verschiedener Initiatoren handeln.

Auch ist die Härtung mit energiereicher Strahlung, z.B. Elektronenstrahlung, möglich.

Auf 100 Gewichtsteile einer Mischung aus den Komponenten a) und b) kommen in der Zusammensetzung vorzugsweise 20 bis 280 Gewichtsteile Füllstoffe.

Auf 100 Gewichtsteile einer Mischung aus den Komponenten a) und b) kommen in der Zusammensetzung vorzugsweise 10 bis 200 Gewichtsteile Glasfasern, Kohlenstofffasern, Aramidfasern, Basaltfasern, Naturfasern oder textile Vliese.

Weitere Zusatzstoffe wie beispielsweise Lichtschutzmittel, schrumpfmindernde thermoplastische Polymere, Eindickmittel, Trennmittel, Hautbildner und Wachse können je nach Verarbeitungsverfahren und Anwendung eingesetzt werden.

Die erfindungsgemäße Harzzusammensetzung, beispielsweise nach Zugabe von Additiven, Fasern und Füllstoffen, kann zur Herstellung von Sheet Molding Compounds (SMC) und Bulk Molding Compounds (BMC) sowie anderer Compounds verwendet werden.

## Verwendung der VE- und VEU-Harze

In einer weiteren Ausführungsform wird die der Erfindung zu Grunde liegende Aufgabe gelöst durch Anwendung der erfindungsgemäßen Harz-Zusammensetzung in einem der folgenden Verarbeitungsverfahren: Beschichten, Lackieren, Gießen, Tauchen, Laminieren, Spaltimprägnieren, Schleudern, Kleben, Harzinjektion, Pressen, Spritzguss, Profilziehen, Spachteln und Wickeln.

In einer weiteren Ausführungsform wird die der Erfindung zu Grunde liegende Aufgabe gelöst durch die Verwendung der erfindungsgemäßen VE- und VEU-Harze und ihrer erfindungsgemäßen Zusammensetzungen z.B. in der Möbelindustrie, in der Medizin und im Gesundheitswesen, in der medizintechnischen Industrie, in Krankenhäusern, Arztpraxen, Altenheimen und Rehabilitationszentren, in der häuslichen Alten- und Krankenpflege, in der Lebensmittel- und fleischerzeugenden, -verarbeitenden und -verpackenden Industrie, in der Verpackungsindustrie, in der Lagerung und Logistik, in der Dichtungsindustrie, in der Tierzucht- und Agrarindustrie und der häuslichen Tierhaltung, in der Pharmaindustrie, in der Haushaltswarenindustrie, im Apparate-, Behälter-, Rohrleitungsbau, in der Elektro-, Kfz- und Bauindustrie, in der Luftfahrtindustrie, in der Textilindustrie, der Hygieneartikelindustrie, in der Sanitärindustrie, in der Sport-, Spielwaren- und Freizeitartikelindustrie, im Schiffsbau, im Bootsbau, der Wassersportindustrie, der Lüftungs- und Klimatechnik, der öffentlichen, häuslichen und industriellen Wasserversorgung sowie der Wasseraufbereitung.

## Herstellung gehärteter Erzeugnisse

In einer weiteren Ausführungsform wird die der Erfindung zu Grunde liegende Aufgabe gelöst durch ein Verfahren zur Herstellung gehärteter Erzeugnisse, wobei man die erfindungsgemäße Zusammensetzung härtet.

Bei der Aushärtung wird die Temperatur vorteilhafterweise auf einen Bereich von 20 bis 200 °C eingestellt, mit Photoinitiatoren sind auch tiefere Temperaturen möglich.

Vorzugsweise wird das gehärtete Erzeugnis, zum Beispiel ein VE- bzw. VEU-Duromer, nach dem erfindungsgemäßen Verfahren zur Herstellung gehärteter Erzeugnisse erhalten. Das Duromer besteht beispielsweise aus dem Vinylester oder Vinylesterurethan und oligomeren Brückenstrukturen, die sich aus dem Reaktivverdünner gebildet haben und die vorzugsweise überwiegend eine mittlere Kettenlänge von 1,5 bis 4 monomeren Reaktivverdünnereinheiten aufweisen.

Der E-Modul des VE- bzw. VEU-Duromers liegt vorteilhafterweise in einem Bereich von 2000 bis 4000 N/mm$^2$. Die Duromere weisen vorteilhafterweise eine Dehnung in einem Bereich von 0,5 bis 6 % auf. Sie sind vorzugsweise geruchsfrei.

Der gehärtete Werkstoff kann faserverstärkt oder unverstärkt sein, mit Füllstoffen gefüllt oder ungefüllt sein und unabhängig davon in technischen Anwendungen der verschiedensten Art, in der Lebensmittelbranche, in Krankenhäusern und bei medizinischen Geräten, in Kühlschränken, Kühlhäusern und vielen anderen Bereichen eingesetzt werden. Das antimikrobielle Verhalten ist eine intrinsische Materialeigenschaft und wird nicht durch konventionelle, additiv zugegebene Biozide bewirkt. Damit unterscheiden sich die erfindungsgemäßen Vinylester- und Vinylesterurethanharz-Zusammensetzungen und die daraus erhaltenen Duromere und Werkstoffe signifikant vom heutigen Stand

der biozid ausgerüsteten Kunststoffe, die üblicherweise mit Nanosilber, Isothiazolinonen, chlororganischen Verbindungen, Triazinderivaten, Verbindungen des Kupfers, Zinns, Zinks und Arsens sowie anderen Wirkstoffen arbeiten. Diese konventionellen Biozide sind wegen einer (meist langsamen) Abgabe an die Umwelt, teilweise schlechter biologischer Abbaubarkeit, eines Schwermetallgehalts, einer möglichen Anreicherung in einzelnen Organismen und/oder einer Verteilung und Verbreitung über die Nahrungsmittelkette umstritten. Mit den neuen intrinsisch antimikrobiellen Harzen und ihren Werkstoffen werden diese Nachteile zuverlässig vermieden.

**Erzeugnis mit antimikrobieller Wirkung**

In einer weiteren Ausführungsform wird die der Erfindung zu Grunde liegende Aufgabe gelöst durch ein Erzeugnis mit antimikrobieller Wirkung enthaltend das erfindungsgemäße ausgehärtete Vinylesterharz oder Vinylesterurethanharz.

Das erfindungsgemäße Erzeugnis besteht zu mehr als 20 Gew.%, vorzugsweise zu mehr als 50 Gew.% und besonders bevorzugt zu mehr als 80 Gew.% aus den Komponenten a) und b).

Das Erzeugnis ist vorzugsweise ein Klebstoff, eine Dichtungsmasse, eine Vergussmasse, eine Beschichtung oder ein Formkörper.

In einer weiteren Ausführungsform wird die der Erfindung zu Grunde liegende Aufgabe gelöst durch die Verwendung der erfindungsgemäßen VE- oder VEU-Harze zur Herstellung der folgenden Produkte:

Möbel und Möbeloberflächen, Kleber, Furniere und Papierlaminate, Knöpfe, Griffe, Tasten, Schalter und Gehäuse, Platten, Fußböden, Rohre, Profile, Tanks und Behälter aller Art insbesondere für Trinkwasser, Lebensmittel und Öle, Auskleidungen aller Art, Dachbeschichtungen, Lichtplatten, Dichtmassen, Kitte, Dübelmassen, Polymerbeton, Agglomarmor, Küchenspülen, Duschtrassen, Badewannen, Waschbecken, Klobrillen, Gartenmöbel, Gartenzäune, Fassadenplatten, Kellerfensterschächte, Fahrzeugteile, Leuchtenträger, Windkraftanlagen, Imprägnierungen, Bindemittel, Vergussmassen, Spachtelmassen und/oder Reaktionsmörtel, Beschichtungen, Lacke, Gelcoats, Topcoats, Schiffe, Boote, Freizeitartikel.

**Verfahren zur Herstellung des aminofunktionalisierten Styrolderivats**

In einer weiteren Ausführungsform wird die der Erfindung zu Grunde liegende Aufgabe gelöst durch ein Verfahren zur Herstellung eines aminofunktionalisierten Styrolderivats, wobei

a) in einem ersten Schritt eine wässrige Alkalihydroxid-Lösung mit einer Konzentration in einem Bereich von 3 bis 7 mol/l (mindestens moläquivalent zur Stoffmenge an Halogenalkyl-Styrol) bereitgestellt wird,

b) in einem zweiten Schritt zu dieser wässrigen Alkalihydroxid-Lösung ein Amin mit wenigstens einem an das Stickstoffatom gebundenen Wasserstoffatom zugefügt wird,

c) in einem dritten Schritt ein Halogenalkyl-Styrol in einer Menge von 0,2 bis 0,75 Moläquivalenten bezogen auf die Menge Amin zugefügt wird,

d) in einem vierten Schritt die entstandene Reaktionslösung nach vollständiger Zugabe des Halogenalkyl-Styrols noch über einen Zeitraum von 4 bis 120 h gerührt wird, und

e) in einem fünften Schritt das entstandene aminofunktionalisierte Styrolderivat von der übrigen Reaktionslösung abgetrennt wird.

Das Halogenalkyl-Styrol ist vorzugsweise am aromatischen Ring in ortho- und/oder meta- und/oder para-Position mit der Halogenalkyl-Gruppe funktionalisiert.

Vorzugsweise wird die wässrige Alkalihydroxid-Lösung mit einer Konzentration in einem Bereich von 4,5-5,5 mol/l bereitgestellt. Die Alkalihydroxid-Lösung ist vorzugsweise eine Natriumhydroxid-Lösung. Diese Lösung hat vorteilhafterweise eine Temperatur in einem Bereich von 20 bis 30 °C.

Das Amin fällt vorteilhafterweise unter die Formel $NHR^1R^2$,
wobei $R^1$ ausgewählt ist aus H, linearen oder verzweigten oder zyklischen Alkylresten aufweisend 1 bis 10 Kohlenstoffatome, und
wobei $R^2$ ein linearer oder verzweigter oder zyklischer Alkylrest aufweisend 1 bis 10 Kohlenstoffatome ist.

Nach vollständiger Zugabe des Amins wird die Reaktionslösung vorzugsweise auf eine Temperatur in einem Bereich von 60 bis 85 °C eingestellt.

Im Halogenalkyl-Styrol ist die Alkylgruppe vorzugsweise mit nur einem Halogenatom substituiert. Die Alkylgruppe ist vorzugsweise Methyl. Das Halogenatom ist vorzugsweise Chlor.

Das Halogenalkyl-Styrol wird vorzugsweise als Lösung in Tetrahydrofuran zugegeben. Die Konzentration der Lösung in Tetrahydrofuran liegt vorzugsweise in einem Bereich von 2 bis 3 mol/l. Vorzugsweise wird das Halogenalkyl-Styrol zu der bisherigen Reaktionslösung zugetropft und die Reaktionslösung vorzugsweise für eine Zeitdauer in einem Bereich von 4 bis 120 h gerührt. Danach findet die Abtrennung vorzugsweise durch Vakuumdestillation statt.

Ein weiterer Gegenstand der vorliegenden Erfindung ist ein Stoff ausgewählt aus

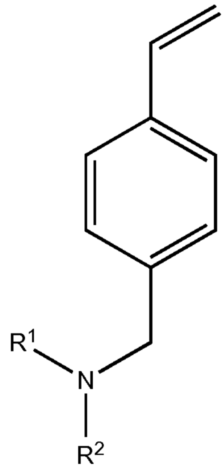
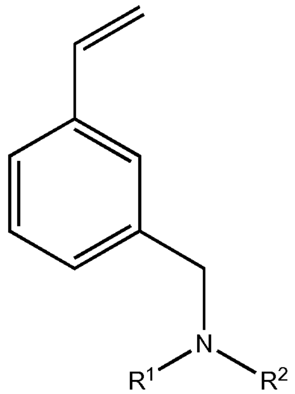
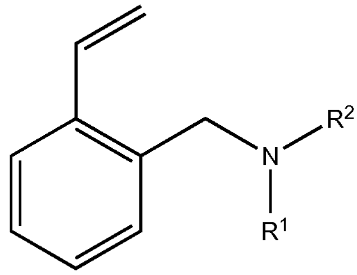

wobei $R^1$ Wasserstoff und $R^2$ tert.-Pentyl ist.

**Verwendung des aminofunktionalisierten Styrolderivats**

In einer weiteren Ausführungsform wird die der Erfindung zu Grunde liegende Aufgabe gelöst durch die Verwendung des erfindungsgemäßen aminofunktionalisierten Styrolderivats zur Herstellung von antimikrobiellen Beschichtungen oder Formkörpern.

**Ausführungsbeispiele**

Herstellung des aminofunktionalisierten Styrolderivats (allgemeine Vorschrift)

In einen 1000 ml Kolben wurden 200 ml Wasser und 42 g (1,05 mol) NaOH gegeben und nach vollständigem Lösen 1,05 mol des entsprechenden Amins zugefügt. Unter Rühren wurde der Kolben auf 60-85 °C aufgeheizt und innerhalb von etwa 75 Minuten eine Lösung aus 53,42 g (0,35 mol) Chlormethylstyrol und 150 ml THF zugetropft.

Nach vollständigem Zutropfen wurde der Reaktionskolben bis zu einer Gesamtreaktionszeit von 4-120 h unter ständigem Rühren im Ölbad belassen. Dabei sind die Reaktionszeit und die Reaktionstemperatur vom verwendeten Amin abhängig. Die Analytik erfolgte mittels GC-MS. Die Aufreinigung erfolgte durch Vakuumdestillation.

Nach dieser allgemeinen Vorschrift wurden aminofunktionalisierte Styrolderivate unter Einsatz folgender Amine synthetisiert: tert.-Butylamin, n-Propylamin, iso-Propylamin, n-Butylamin, sec-Butylamin, iso-Butylamin, n-Pentylamin, 3-Pentylamin, iso-Pentylamin, tert.-Pentylamin, Cyclopentylamin, Cyclohexylamin, Diethylamin, Diisopropylamin.

Das mit tert.-Butylamin erhaltene tert.-Butylaminomethylstyrol erhielt die Abkürzung TBAMS. Es wurde bei einer Reaktionstemperatur von 70 °C und einer Nachrührzeit von 24 h mit einem Umsatz von > 98% und einer Selektivität von > 98% hergestellt. Bei der Vakuumdestillation war der Siedepunkt von TBAMS 115 °C bei 6 mbar.

**Beispiel 1**

In eine Braunglasflasche wurden 44,26 g (35 Gew.-%) des Bisphenol A-basierten Vinylesters CRYSTIC VE 671 der Fa. Scott Bader und 82,29 g (65 Gew.-%) des aminofunktionalisierten Reaktivverdünners TBAMS (tert.-Butylaminomethylstyrol) zum Lösen 14 Tage auf einen Rollbock gelegt und nach vollständigem Lösen mit 2 Gew.-% des Azoinitiators V601 [Dimethyl 2,2'-azobis(2-methylpropionat)] der Fa. Wako versetzt. Anschließend wurden jeweils etwa 8 g der transparenten homogenen Harz-Initiator-Mischung auf verschiedene Glaspetrischalen gegeben und unter Stickstoffatmosphäre bei 70 °C für 2 h, bei 80 °C für 2 h und bei 90 °C für 2h gehärtet.

Das erhaltene, voll ausgehärtete VE-Duromer war klebfrei und hart. Das VE-Duromer war praktisch geruchsfrei und zeigte exzellente antimikrobielle Eigenschaften.

**Beispiel 2**

In einen Dreihalskolben wurden 0,25 g (600 ppm) 3,5 Di-tert.-butyl-4-hydroxyltoluol (BHT), 0,03 g (60 ppm) 4-Methoxyphenol (HQME), 57,68 g (0,4 mol) 2-Hydroypropylmethacrylat (HPMA), 32,30 g (0,2 mol) tert.-Butyl-bis-hydroxyethylamin (TBBHEA) und 84,90 g (20 Gew.-% bezogen auf das vollständige VEU-Harz) Diethylaminomethylstyrol (DEAMS) eingewogen. Die Mischung wurde unter ständigem Rühren auf 65 °C erwärmt. Nach Erreichen der Reaktionstemperatur wurden 101,08 g (0,404 mol) Lupranat MI der BASF SE (49 Gew.-% 4,4'-Methylendiphenyldiisocyanat, 49 Gew.-% 2,4'-Methylendiphenyldiisocyanat, 2 Gew.-% 2,2'-Methylendiphenyl-diisocyanat) so zugetropft, dass die Reaktionsmischung eine konstante Temperatur zwischen 70°C und 80 °C erreichte. Die entstehende Wärme wurde über ein Wasserbad abgeführt. Nach vollständigem Zutropfen (40 min) wurde die Mischung 1 Stunde nachgerührt. Anschließend wurden 148,75 g (35 Gew.-% bezogen auf das vollständige VEU-Harz) tert.-Butylaminomethylstyrol (TBAMS) hinzugefügt und weitere 5 Minuten gerührt.

Zur Härtung wurden 2 Gew.-% des Azoinitiators V601 [Dimethyl 2,2'-azobis(2-methylpropionat)] der Fa. Wako zugesetzt und im Harz vollständig gelöst. Anschließend wurden jeweils etwa 8 g der transparenten homogenen Harz-Initiator-Mischung auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete VEU-Harz-Duromer war hart, klebfrei, praktisch geruchsfrei und zeigte exzellente antimikrobielle Eigenschaften.

In diesem Ausführungsbeispiel beträgt das Verhältnis von Styrolderivat zu Doppelbindung 3,07 : 1.

**Beispiel 3**

In einen Dreihalskolben wurden 0,25 g (600 ppm) 3,5 Di-tert.-butyl-4-hydroxyltoluol (BHT), 0,03 g (60 ppm) 4-Methoxyphenol (HQME), 57,68 g (0,4 mol) 2-Hydroypropylmethacrylat (HPMA), 32,30 g (0,2 mol) tert.-Butyl-bis-hydroxyethylamin (TBBHEA) und 200 ml wasserfreies Aceton gegeben. Die Mischung wurde unter ständigem Rühren auf 65 °C erwärmt. Nach Erreichen der Reaktionstemperatur wurden 101,08 g (0,404 mol) Lupranat MI der BASF SE (49 Gew.-% 4,4'-Methylen-diphenyldiisocyanat, 49 Gew.-% 2,4'-Methylendiphenyldiisocyanat, 2 Gew.-% 2,2'-Methylendiphenyl-diisocyanat) so zugetropft, dass die Reaktionsmischung eine konstante Temperatur zwischen 65 °C und 75 °C erreichte. Die entstehende Wärme wurde über ein Wasserbad abgeführt. Nach vollständigem Zutropfen (25 Minuten) wurde die Mischung 1 Stunde nachgerührt. Anschließend wurde der Kolbeninhalt etwa gleichmäßig auf drei leer gewogene Einhalskolben verteilt und in jeden der Kolben eine Menge von 20,00 g tert.-Butylaminomethylstyrol (TBAMS) gegeben. Anschließend wurde das Aceton aus allen Kolben erst bei Normaldruck und anschließend im Vakuum bis zu einer konstant bleibenden Kolbengesamtmasse abgetrennt. Nach Rückwiegen wurde die im jeweiligen Kolben vorhandene VEU-Masse errechnet.

**Beispiel 3A.**

Herstellung und Härtung des VEU-Harzes mit 65 Gew.% TBAMS

Zum vorhandenen Kolbeninhalt von 66,09 g VEU und 20,00 g TBAMS wurden weitere 102,40 g TBAMS hinzugefügt, um ein VEU-Harz mit einem TBAMS-Gesamtgewichtsanteil von 65 % herzustellen. Anschließend wurden 2 Gew.-% des Azoinitiators V601 [Dimethyl 2,2'-azobis(2-methylpropionat)] der Fa. Wako zugesetzt und der Kolbeninhalt homogenisiert. Jeweils etwa 8 g der transparenten homogenen Harz-Initiator-Mischung wurden auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete VEU-Harz-Duromer war hart, klebfrei, praktisch geruchsfrei und zeigte exzellente antimikrobielle Eigenschaften.

In diesem Ausführungsbeispiel soll exemplarisch die Berechnung des Verhältnisses Styrolderivat zu Doppelbindung veranschaulicht werden.

Der Vinylesterurethananteil (VEU) entspricht Komponente a) und beträgt 66,09 g (35 Gew.-%). Aus obenstehendem Beispiel 3 kann man sehen, dass diese Komponente a) aus den Additionsreaktionen von einem Äquivalent Kettenverlängerer (tert.-Butyl-bis-hydroxyethylamin) mit zwei Äquivalenten Diisocyanat (Methylenphenyldiisocyanat) und zwei Äquivalenten eines 2-Hydroxypropylmethacrylat gewonnen wird, sodass ihr Molgewicht 950 g/mol beträgt. Die VEU-Komponente weist zwei vinylische Doppelbindungen auf; also ist die Funktionalität f gleich 2.

Somit ergibt sich $n_{Dop}=f \cdot m_{Dop}/M_{Dop}=2 \cdot 66{,}09 g/950 g/mol=0{,}139 mol$
Der Styrolderivatanteil (tert.Butylaminomethylstyrol, TBAMS) entspricht Komponente b) und beträgt 20 +102,4g= 122,4g (65 Gew.-%). Das Molgewicht beträgt 189 g/mol.

Somit ergibt sich $n_{Sty}=m_{Sty}/M_{Sty}=122{,}4 g/189 g/mol=0{,}648 mol$
Das Verhältnis von Styrolderviat zu Doppelbindung beträgt:

$n_{Sty}$: $n_{Dop}$= 0,648 mol:0,139mol= 4,66 : 1

**Beispiel 3B**

Herstellung und Härtung des VEU-Harzes mit 60 Gew.% TBAMS

Zum vorhandenen Kolbeninhalt von 58,73 g VEU und 20,00 g TBAMS wurden weitere 68,10 g TBAMS hinzugefügt, um ein VEU-Harz mit einem TBAMS-Gesamtgewichtsanteil von 60 % herzustellen. Anschließend wurden 2 Gew.-% des Azoinitiators V601 [Dimethyl 2,2'-azobis(2-methylpropionat)] der Fa. Wako zugesetzt und der Kolbeninhalt homogenisiert. Jeweils etwa 8 g der transparenten homogenen Harz-Initiator-Mischung wurden auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete VEU-Harz-Duromer war hart, klebfrei, praktisch geruchsfrei und zeigte exzellente antimikrobielle Eigenschaften.

In diesem Ausführungsbeispiel beträgt das Verhältnis von Styrolderivat zu Doppelbindung 3,79 : 1.

**Beispiel 3C**

Herstellung und Härtung des VEU-Harzes mit 55 Gew.% TBAMS

Zum vorhandenen Kolbeninhalt von 57,43 g VEU und 20,00 g TBAMS wurden weitere 50,19 g TBAMS hinzugefügt, um ein VEU-Harz mit einem TBAMS-Gesamtgewichtsanteil von 55 % herzustellen. Anschließend wurden 2 Gew.-% des Azoinitiators V601 [Dimethyl 2,2'-azobis(2-methylpropionat)] der Fa. Wako zugesetzt und der Kolbeninhalt homogenisiert. Jeweils etwa 8 g der transparenten homogenen Harz-Initiator-Mischung wurden auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete VEU-Harz-Duromer war hart, klebfrei, praktisch geruchsfrei und zeigte exzellente antimikrobielle Eigenschaften.

In diesem Ausführungsbeispiel beträgt das Verhältnis von Styrolderivat zu Doppelbindung 3,07 :1.

**Beispiel 4**

In eine Braunglasflasche wurden 50,00 g (37,6 Gew.-%) des Vinylesters (1-methyl-ethylidene)bis[4,1-phenyleneoxy(2-hydroxy-3,1-propanediyl)]bismeth-acrylate (entspricht Komponente a) erhältlich bei Sigma Aldrich, Produktnummer: 494356, M=512,59 g/mol) und 83,09 g (62,4 Gew.-%) des aminofunktionalisierten Reaktivverdünners TBAMS (tert.-Butylaminomethylstyrol entspricht Komponente b)) zum Lösen 4 Tage auf einen Rollbock gelegt und nach vollständigem Lösen mit 2 Gew.-% des Azoinitiators V601 [Dimethyl 2,2'-azobis(2-methylpropionat)] der Fa. Wako versetzt. Anschließend wurden jeweils etwa 8 g der transparenten homogenen Harz-Initiator-Mischung auf verschiedene Glaspetrischalen gegeben und unter Stickstoffatmosphäre bei 70 °C für 2 h, bei 80 °C für 2 h und bei 90 °C für 2h gehärtet.

Das erhaltene, voll ausgehärtete VE-Duromer war klebfrei und hart. Das VE-Duromer war praktisch geruchsfrei und zeigte exzellente antimikrobielle Eigenschaften.

In diesem Ausführungsbeispiel beträgt Verhältnis von Styrolderivat zu Doppelbindung beträgt 2,26 :1.

**Vorgehen zur Bestimmung des mechanischen Verhaltens und der Glasübergangstemperatur**

Zur Bestimmung der Netzwerk-TG wurden aus den Harzen glasfaserverstärkte Duromer-Probekörper hergestellt und mittels dynamisch-mechanischer Analyse (DMA) charakterisiert.

Die verwendete DMA 242 der Fa. Netzsch ermöglicht die Bestimmung des Speicher- und Verlustmoduls, sowie des Verlustfaktors einer Probe als Funktion der Temperatur, Zeit und Frequenz einer aufgebrachten sinusförmigen Schwingungsbelastung.

Zur Herstellung der benötigten Probekörper wurden die in den Ausführungsbeispielen hergestellten, mit 2 Gew.-% Initiator (V601 Fa. Wako) versetzten Harze verwendet.

Hierzu wurden drei 15x15 cm Lagen Saertex®-Glasfasergelege (biaxial 0°/90°/ Typ: S14EB540-00620-T1300-487000) mit dem jeweiligen Harz imprägniert, in eine verschraubbare, mit Mylar®-Folie ausgekleidete 150x150x5 mm Plattenform gelegt und durch Andrücken mit einem Spatel möglichst von Luftblasen befreit. Anschließend wurde die Kavität der Form mit weiterem Harz vollständig gefüllt, mit Mylar®-Folie abgedeckt und mit der oberen Werkzeugplatte durch Verschraubung verschlossen. Die Härtung erfolgte für jeweils 2 h bei 70, 80 und 90 °C in einem Trockenschrank.

Nach Abkühlen wurden die GFK-Platten mit einer Tischkreissäge zugeschnitten und ggf. mit einem Schleifband auf die erforderliche Probengröße gebracht.

Parameter und Messeinstellungen bei den durchgeführten DMA-Analysen:

Probenabmessungen: 50 x 10 x 5 mm

Deformationsmodus: Zweiarmige Biegung

Amplitude: 30 μm

Dynamische Kraft: 7,55 N

Statische Kraft: 4 N

Temperaturbereich: 20 - 160 °C

Heizrate: 2 K/min

Frequenz: 1 Hz / 10 Hz

Atmosphäre: N2

Flussrate N2: 5 ml/min

**Vorgehen bei den antimikrobiellen Untersuchungen**

Die angewendete Methode basierte auf dem japanischen Standard JIS Z 2801:2000. Testmikroorganismus für die Versuche war der pathogene Keim Staphylococcus aureus. Es wurde ein Standardkeim (ATCC 6538) verwendet - nicht ein multiresistenter.

Bei jedem Testkeim (hier Staphylococcus aureus) stellte man unter den Bedingungen der Herstellung der Ausgangslösung oder Ausgangssuspension einen Mikroorganismus-spezifischen Keimgehalt ein. Bei Staphylococcus aureus betrug dieser Wert 108 Keime pro ml (siehe auch Ausführungen weiter unten).

Die Bestimmung der antimikrobiellen Aktivität erfolgte durch den Vergleich des Wachstums von Staphylococcus aureus auf Referenzoberflächen und auf den Probenmaterialien.

Als Referenzmaterial dienten leere Petrischalen. Die Proben bestanden aus Petrischalen, in die jeweils eine dünne Schicht einer Polymerprobe gegossen war. Für jede Versuchsreihe wurden drei Referenzplatten zur Bestimmung des Anfangskeimgehaltes (separater Versuch unabhängig von der Untersuchung des antimikrobiellen Verhaltens) sowie drei Referenzplatten und drei Probenplatten zur Bestimmung des Oberflächenkeimgehaltes nach der Inkubation herangezogen.

Alle Platten wurden mit 400 μl Staphylococcus aureus Impfsuspension beimpft, die auf einen Keimgehalt von 4,0 - 10 * $10^5$ KbE/ml eingestellt war.

Die aufgebrachte Impfsuspension wurde mit einer sterilen PP-Folie abgedeckt, um Verdunstung zu vermeiden. Unmittelbar nach dem Beimpfen wurden die drei Probenplatten und drei Referenzplatten in einen Inkubationsschrank überführt und bei 35°C und 90% Luftfeuchte für 2 h bzw. 24 h gelagert.

Zur Bestimmung des Keimgehaltes in der Beimpfungslösung (Anfangskeimgehalt) wurden je drei Referenzplatten direkt nach dem Beimpfen, unter Zugabe von 10 ml SCDLP-Bouillon (Soja-Casein-Pepton-Bouillon mit Lecithin und Polyoxyethylenmedium) in die Petrischale, ausgewaschen. Die Folie wurde mit einer sterilen Pinzette gewendet und mehrmals mit einer 1 ml - Pipette über- beziehungsweise unterspült. Die Petrischale wurde in Form einer Acht geschwenkt, bevor 1 ml der Auswaschlösung in die erste Verdünnungsstufe pipettiert wurde. Nach Anlegen einer Verdünnungsreihe wurde der Lebendkeimgehalt mittels Drop-Plate-Verfahren bestimmt. Beim Drop-Plate-Verfahren wurden - im Doppelansatz - auf einer Plate-Count-(PC)-Agar-Platte in jeden Sektor der jeweiligen Verdünnungsstufe 5 Tropfen je 10 μl aufgebracht. Die Bebrütung der Platten erfolgte für 2 h bzw. 24 h bei 37°C.

Das Auswaschen und die Bestimmung der Lebendkeimzahl auf den Referenz- und Probenplatten nach der Inkubation erfolgten in gleicher Art und Weise wie die Bestimmung des Anfangskeimgehaltes. Bei den Probenplatten wurde zusätzlich zur Erhöhung der Nachweisgrenze der Keimgehalt der direkten Auswaschlösung im Plattengussverfahren bestimmt. Hierzu wurde, ebenfalls im Doppelansatz, je 1 ml der Lösung in eine leere Petrischale gegeben und mit flüssigem, auf 45 °C temperiertem PC-Agar übergossen. Durch Schwenken in Form einer Acht wurden die Bakterien im Agar verteilt. Die Bebrütung der Platten erfolgte für 48 h bei 37 °C.

Nach der Inkubation wurden die Kolonien in der Petrischale gezählt. Dabei wurde unterstellt, dass aus jedem Keim eine sichtbare Kolonie geworden war. Die Kolonien waren nach der Bebrütung mit bloßem Auge sichtbar - zur Hilfe konnte ein Lichttisch herangezogen werden, um die Keime besser zu erkennen.

Anhand des Volumens der Beimpfungslösung und der verwendeten Verdünnungsverhältnisse konnte auf die

Lebendkeimzahl der Mikroorganismen pro Volumeneinheit (d.h. pro ml) Beimpfungslösung rückgeschlossen werden. Die Berechnung erfolgte anhand des gewogenen arithmetischen Mittels mit folgender Formel:

$$\bar{c} = \frac{\sum c}{n_1 \cdot 1 + n_2 \cdot 0{,}1} \cdot d$$

Mit

$\bar{c}$ gewogenes arithmetisches Mittel

$\sum_c$ Summe der Kolonien aller Petrischalen oder Sektoren, die zur Berechnung herangezogen werden

$n_1$ Anzahl der Petrischalen oder Sektoren der niedrigsten auswertbaren Verdünnungsstufe

$n_2$ Anzahl der Petrischalen oder Sektoren der nächst höheren Verdünnungsstufe

$d$ Faktor der niedrigsten ausgewerteten Verdünnungsstufe

Beim Plattengussverfahren waren Petrischalen mit bis zu 300 KbE zählbar. Beim Drop-Plate-Verfahren waren nur Platten bis 150 KbE pro Sektor auswertbar.

Bei der Bestimmung der Lebendkeimzahl pro ml musste der Verdünnungsfaktor *F*1 beachtet werden. Dieser ergab sich aus der Summe des Volumens der SCDLP-Bouillon und dem Volumen der Bakteriensuspension auf der beimpften Platte dividiert durch das Volumen der Bakteriensuspension auf dieser beimpften Platte.

$$F_1 = \frac{10\,ml + 0{,}4\,ml}{0{,}4\,ml} = 26$$

$F_1$ Verdünnungsfaktor der SCDLP-Bouillon

Für die Gesamtkeimzahl auf den beimpften Proben- beziehungsweise Referenzplatten im Plattengussverfahren ergab sich somit folgende Formel:

$$KbE = \frac{\sum c}{n_1 \cdot 1 + n_2 \cdot 0{,}1} \cdot d \cdot F_1$$

Beim Drop-Plate-Verfahren war noch ein weiterer Verdünnungsfaktor relevant, da ein Viertel einer Platte nur mit 50 µl, also 0,05 ml, beimpft wurde. Um auf den Keimgehalt pro ml zu schließen, mussten 0,05 ml auf 1 ml umgerechnet werden, indem man mit 20 multiplizierte.

$$F_2 = 26 \cdot 20$$

$F_2$ Verdünnungsfaktor, um die KbE beim Drop-Plate-Verfahren pro ml zu erhalten

Entsprechend wurde die Gesamtkeimzahl der beimpften Proben- beziehungsweise der Referenzplatten für das Drop-Plate-Verfahren unter Berücksichtigung aller Verdünnungsfaktoren nach folgender Formel berechnet:

$$KbE = \frac{\sum c}{n_1 \cdot 1 + n_2 \cdot 0{,}1} \cdot d \cdot F_2$$

Für die Berechnung der antimikrobiellen Aktivität wurden in jeder Versuchsreihe die Einzelergebnisse der Lebendkeimzahl für die Platten als einfaches arithmetisches Mittel zusammengefasst und daraus die $Log_{10}$-Reduktion zwischen Proben und Referenzplatten ermittelt.

Die Berechnung erfolgte mittels nachstehender Formel:

$$\log_{10}-Reduktion = \log_{10}(KG)_{Ref(x)} - \log_{10}(KG)_{Pr(x)}$$

wobei

$(KG)_{Ref(x)}$ KbE auf den Referenzplatten zum Zeitpunkt x

$(KG)_{Pr(x)}$ KbE auf den Probenplatten zum Zeitpunkt x

Laut JIS Z 2801:2000 ist eine antimikrobielle Aktivität bei einer Log-Reduktion von mindestens 2,0 nach 24 Stunden Einwirkzeit gegeben.

Waren auf den Agar-Platten der Proben bei der niedrigsten Verdünnungsstufe im Plattengussverfahren keine auszählbaren Kolonien vorhanden, wurde das Ergebnis mit <10 KbE/ml angegeben entsprechend der Vorgabe im Teststandard.

Ergebnisse der mechanischen und antimikrobiellen Untersuchungen:

| Beispiel | Zusammensetzung VE bzw. VEU | Reaktivverdünner (Massenanteil) | $T_G$ bei 1 Hz | $T_G$ bei 10 Hz | Anfangskeimgehalt (log KbE/ml) | Referenzkeimgehalt nach 24 Stunden (log KbE/ml) | Oberflächenkeimgehalt Probe nach 24 Stunden (log KbE/ml) | Log-Reduktion |
|---|---|---|---|---|---|---|---|---|
| 1 | Crystic VE 671 (Scott Bader) | TBAMS (0,65) | 104,3 | 111,5 | 5,6 | 7,5 | 1,1 | 6,4 |
| 2 | $HPMA_{0,4}TBBHEA_{0,2}$ Lupranant $MI_{0,404}$ | DEAMS (0,2) / TBAMS (0,35) | 106,7 | 114,8 | 5,3 | 8,4 | 2,7 | 5,7 |
| 3A | $HPMA_{0,4}TBBHEA_{0,2}$ Lupranant MIo.aoa | TBAMS (0,65) | 97,6 | 106,4 | 5,3 | 8,4 | 1 | 7,4 |
| 3B | $HPMA_{0,4}$ $TBBHEA_{0,2}$ Lupranant$MI_{o\cdot 404}$ | TBAMS (0,60) | 102,6 | 111,2 | 5,3 | 8,4 | 3,1 | 5,3 |
| 3C | $HPMA_{0,4}TBBHEA_{0,2}$ Lupranant $MI_{o\cdot aoa}$ | TBAMS (0,55) | 100,5 | 109,1 | 5,3 | 8,4 | 2,4 | 6,0 |
| 4 | (1-Methylethyliden)bis-[4,1-phenylen-oxy(2-hydroxy-3,1-propanediyl)]bis methacrylat | TBAMS (0,62) | 110,3 | 117,5 | 5,7 | 7,8 | 1,5 | 6,3 |

## Patentansprüche

1. Harz-Zusammensetzung zur Herstellung von Erzeugnissen mit antimikrobieller Wirkung enthaltend

   a) Vinylester und/oder Vinylesterurethan, und
   b) Styrolderivat als Reaktivverdünner,

   wobei je Doppelbindung in Komponente a) 0,5 bis 8 Styrolderivatmoleküle in der Zusammensetzung vorhanden sind, wobei die Komponente b) aminofunktionalisiert ist und die Aminofunktionalität unter die Formel

   $$-(CH_2)_q-NH_pR^1R^2A_p$$

   fällt, wobei

   q entweder 1 oder 2 ist,
   p 0 oder 1 ist,
   $R^1$ ausgewählt ist aus H, linearen oder verzweigten oder zyklischen Alkylresten aufweisend 1 bis 10 Kohlenstoffatome,
   $R^2$ ein linearer oder verzweigter oder zyklischer Alkylrest aufweisend 1 bis 10 Kohlenstoffatome ist,
   A das Anion einer Säure ist und,
   der Amin-Stickstoff N der obenstehenden Formel neutral (p = 0) oder positiv (p = 1) geladen ist.

2. Zusammensetzung gemäß Anspruch 1, **dadurch gekennzeichnet, dass** das Vinylesterurethan aminogruppenhaltige Kettenverlängerer enthält.

3. Zusammensetzung gemäß der Ansprüche 1 oder 2, **dadurch gekennzeichnet, dass** das aminofunktionalisierte Styrolderivat einer dieser Stoffe ist

   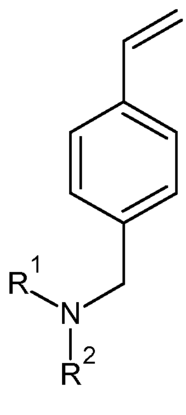
   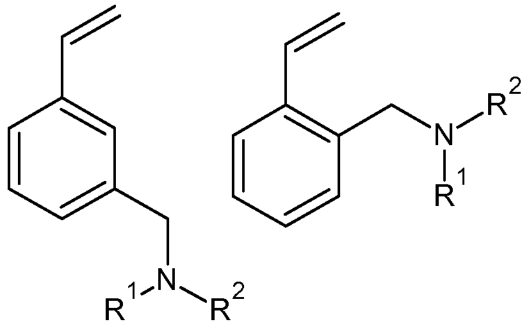

   wobei $R^1$ Wasserstoff ist und
   wobei $R^2$ ausgewählt ist aus der Gruppe iso-Propyl, tert.-Butyl, tert.-Pentyl.

4. Zusammensetzung gemäß einem der Ansprüche 1 bis 3, **dadurch gekennzeichnet, dass** sie wenigstens 20 Gew.%, insbesondere wenigstens 80 Gew.% einer Mischung der Komponenten a) und b) enthält.

5. Zusammensetzung gemäß einem der Ansprüche 1 bis 4, wobei je Doppelbindung in Komponente a) 1,5 bis 4 Styrolderivatmoleküle in der Zusammensetzung vorhanden sind.

6. Verwendung der Zusammensetzung gemäß einem der Ansprüche 1 bis 5 in einem der folgenden Verarbeitungsverfahren: Beschichten, Lackieren, Gießen, Tauchen, Laminieren, Spaltimprägnieren, Schleudern, Kleben, Harzinjektion, Pressverfahren, Spritzguss, Profilziehen, Spachteln und Wickeln.

7. Verfahren zur Herstellung gehärteter Erzeugnisse, wobei man eine Zusammensetzung gemäß einem der Ansprüche 1 bis 5 härtet.

**8.** Erzeugnis mit antimikrobieller Wirkung enthaltend eine gehärtete Zusammensetzung gemäß Anspruch 7.

**9.** Verwendung des Erzeugnisses nach Anspruch 8 zur Herstellung der folgenden Produkte: Möbel und Möbeloberflächen, Kleber, Furniere und Papierlaminate, Knöpfe, Griffe, Tasten, Schalter und Gehäuse, Platten, Fußböden, Rohre, Profile, Tanks und Behälter, insbesondere für Trinkwasser, Lebensmittel und Öle, Auskleidungen, Dachbeschichtungen, Lichtplatten, Dichtmassen, Kitte, Dübelmassen, Polymerbeton, Agglomarmor, Küchenspülen, Duschtrassen, Badewannen, Waschbecken, Klobrillen, Gartenmöbel, Gartenzäune, Fassadenplatten, Kellerfensterschächte, Fahrzeugteile, Leuchtenträger, Windkraftanlagen, Imprägnierungen, Bindemittel, Vergussmassen, Spachtelmassen und/oder Reaktionsmörtel, Beschichtungen, Lacke, Gelcoats, Topcoats, Schiffe, Boote, Freizeitartikel.

**10.** Verfahren zur Herstellung eines aminofunktionalisierten Styrolderivats, wobei

a) in einem ersten Schritt eine wässrige Alkalihydroxid-Lösung mit einer Konzentration in einem Bereich von 3 bis 7 mol/l bereitgestellt wird,
b) in einem zweiten Schritt zu dieser wässrigen Alkalihydroxid-Lösung eine halbmoläquivalente bis moläquivalente Menge an einem Amin mit wenigstens einem an das Stickstoffatom gebundenen Wasserstoffatom zugefügt wird,
c) in einem dritten Schritt ein Halogenalkyl-Styrol in einer Menge von 0,2 bis 0,5 Moläquivalenten bezogen auf die Menge Alkalihydroxid zugefügt wird,
d) in einem vierten Schritt die entstandene Reaktionslösung nach vollständiger Zugabe des Halogenalkyl-Styrol noch über einen Zeitraum in einem Bereich von 2 bis 48 h gerührt wird, und
e) in einem fünften Schritt das entstandene aminofunktionalisierte Styrolderivat von der übrigen Reaktionslösung abgetrennt wird.

**11.** Verwendung eines oder einer Mischung dieser drei Stoffe ausgewählt aus

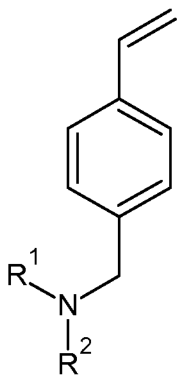
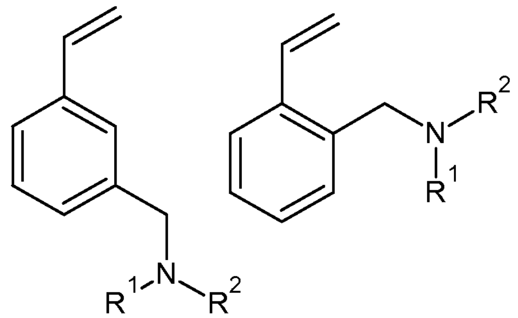

als Reaktivverdünner in Harzanwendungen,
wobei $R^1$ und/oder $R^2$ unabhängig ausgewählt sind aus der Gruppe Methyl, Ethyl, n-Propyl, iso-Propyl, n-Butyl, sec-Butyl, iso-Butyl, tert.-Butyl, n-Pentyl, 3-Pentyl, iso-Pentyl, tert.-Pentyl, Cyclopentyl, Cyclohexyl,
wobei $R^1$ auch H sein kann.

**12.** Stoff ausgewählt aus

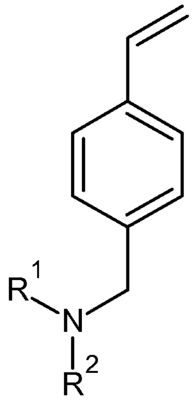
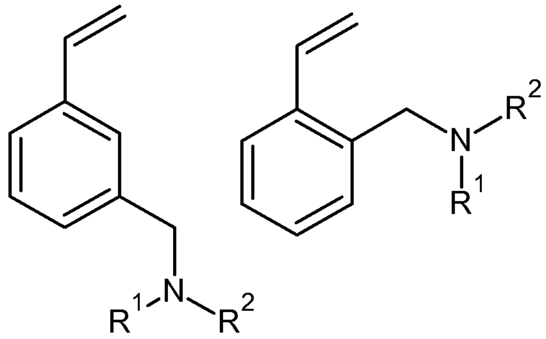

wobei $R^1$ Wasserstoff und $R^2$ tert.-Pentyl ist.

**13.** Verwendung des aminofunktionalisierten Styrolderivats gemäß Anspruch 11 oder 12 oder erhalten gemäß Anspruch 10 zur Herstellung von antimikrobiellen Beschichtungen oder Formkörpern.

## Claims

**1.** A resin composition for the preparation of products having an antimicrobial effect, containing

a) vinyl ester and/or vinyl ester urethane, and
b) styrene derivative as a reactive diluent,

wherein for each double bond in component a) there are 0.5 to 8 styrene derivative molecules present in the composition,
wherein component b) is amino-functionalized and the amino functionality is of the formula

$$-(CH_2)_q-NH_pR^1R^2A_p,$$

wherein

q is either 1 or 2,
p is 0 or 1,
R is selected from H, linear or branched or cyclic alkyl radicals comprising 1 to 10 carbon atoms,
$R^2$ is a linear or branched or cyclic alkyl radical comprising 1 to 10 carbon atoms,
A is the anion of an acid, and
the amine nitrogen N of the above formula is neutrally (p = 0) or positively (p = 1) charged.

**2.** A composition according to claim 1, **characterized in that** the vinyl ester urethane contains amino-group-containing chain extenders.

**3.** A composition according to claim 1 or 2, **characterized in that** the amino-functionalized styrene derivative is one of these substances

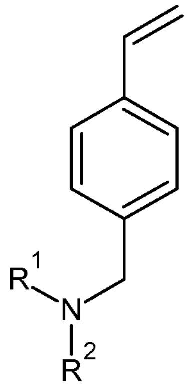
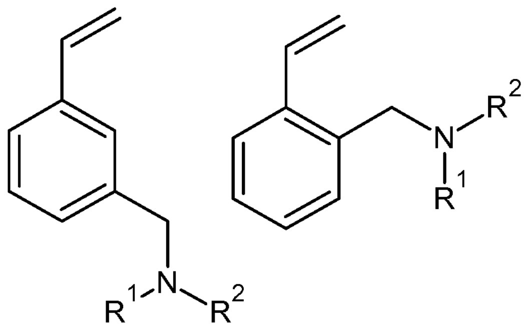

wherein $R^1$ is hydrogen and
wherein $R^2$ is selected from the group of isopropyl, tert. butyl, tert. pentyl.

**4.** A composition according to any of claims 1 to 3, **characterized in that** it contains at least 20 wt %, especially at least 80 wt %, of a mixture of components a) and b)

**5.** A composition according to any of claims 1 to 4, wherein for each double bond in component a) there are 1.5 to 4 styrene derivative molecules present in the composition.

**6.** A use of the composition according to any of claims 1 to 5 in one of the following processing procedures: Coating, painting, pouring, dipping, laminating, gap impregnation, spinning, gluing, resin injection, compression moulding, injection moulding, profile drawing, filling and wrapping.

**7.** A method for the preparation of cured products, wherein a composition according to any of claims 1 to 5 is cured.

**8.** A product having an antimicrobial effect including a cured composition according to claim 7.

**9.** A use of the product according to claim 8 in the preparation of the following products: Furniture and furniture coatings, adhesives, veneers and paper laminates, knobs, handles, buttons, switches and housings, panels, floors, pipes, profiles, tanks and containers, in particular for drinking water, food and oils, linings, roof coatings, light panels, sealants, cements, plugging compounds, polymer concrete, agglomarble, kitchen sinks, shower trays, bathtubs, sinks, toilet seats, garden furniture, garden fences, facade panels, basement window wells, vehicle parts, light carriers, wind power plants, impregnations, binders, sealing compounds, fillers and/or reaction mortar, coatings, varnishes, gel coats, top coats, ships, boats, recreational items.

**10.** A method for the preparation of the amino-functionalized styrene derivative, wherein,

a) in a first step, an aqueous alkali hydroxide solution is provided in a concentration ranging from 3 to 7 mol/l,
b) in a second step, in an amount of half a molar equivalent to a molar equivalent, an amine having at least one hydrogen atom bonded to the nitrogen atom is added to this aqueous alkali hydroxide solution,
c) in a third step, a haloalkyl styrene is added in an amount of 0.2 to 0.5 molar equivalents based on the amount of alkali hydroxide,
d) in a fourth step, once all the haloalkyl styrene has been added, the resulting reaction solution is stirred over a period of another 2 to 48 hours, and
e) in a fifth step, the formed amino-functionalized styrene derivative formed is separated from the rest of the reaction solution.

**11.** A use of one or a mixture of these three substances selected from

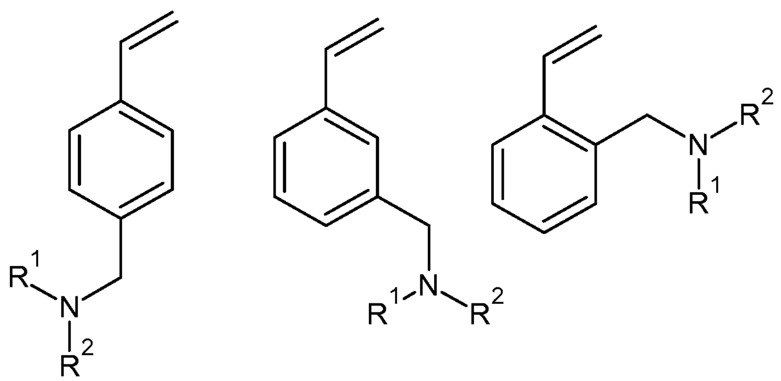

as a reactive diluent in resin applications,
wherein $R^1$ and/or $R^2$ are independently selected from the group of methyl, ethyl, n-propyl, isopropyl, n-butyl, sec. butyl, isobutyl, tert. butyl, n-pentyl, 3-pentyl , isopentyl, tert. pentyl, cyclopentyl, cyclohexyl,
wherein $R^1$ may also be H.

**12.** A substance selected from

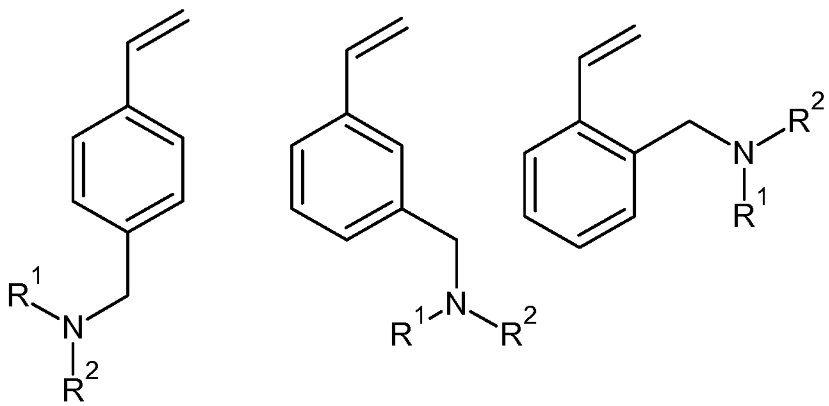

wherein $R^1$ is hydrogen and $R^2$ is tert. pentyl.

**13.** A use of the amino-functionalized styrene derivative according to claim 11 or 12 or obtained according to claim 10 in the preparation of antimicrobial coatings or mouldings.

**Revendications**

**1.** Composition de résine pour la fabrication de produits à activité antimicrobienne comprenant

a) un ester vinylique et / ou un ester vinylique d'uréthane et
b) un dérivé du styrène en tant que diluant réactif,

pour chaque double liaison dans le composant a) de 0,5 à 8 molécules de dérivé du styrène étant présents dans la composition,
le composant b) étant muni d'une fonction amino et la fonction amino est représenté par la formule

$$-(CH_2)_q-NH_pR^1R^2A_p$$

dans laquelle

q est soit 1 ou 2,
p est égal à 0 ou 1,

$R^1$ est choisi parmi H, les radicaux alkyles comprenant de 1 à 10 atomes de carbone, linéaire ou ramifié ou cyclique,
$R^2$ est une radical alkyle linéaire, ramifié ou cyclique comprenant de 1 à 10 atomes de carbone,
A représente l'anion d'un acide, et
l'azote aminé N de la formule ci-dessus est chargé neutre (p = 0) ou positif (p = 1).

**2.** Composition selon la revendication 1, **caractérisée en ce que** l'ester vinylique d'uréthane contient des agents d'allongement de chaîne contient des groupes amino.

**3.** Composition selon les revendications 1 ou 2, **caractérisé en ce que** le dérivé du styrène à fonctionnalité amino est une de ces substances

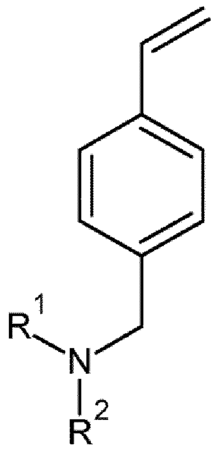

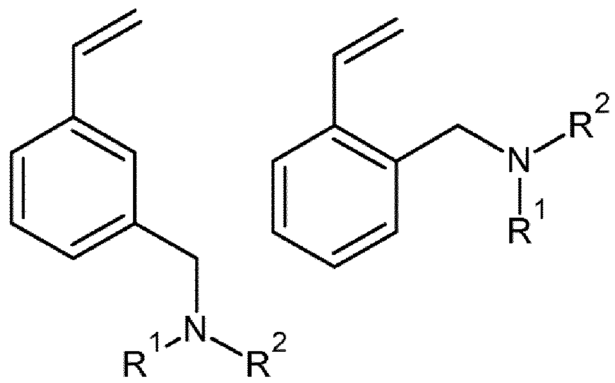

dans laquelle $R^1$ est un atome d'hydrogène et
dans laquelle $R^2$ est choisi parmi le groupe isopropyle, tert-butyle, tert-pentyle.

**4.** Composition selon l'une quelconque des revendications 1 à 3, **caractérisé en ce qu'**elle contient au moins 20 % en poids, en particulier au moins 80 % en poids, d'un mélange des composants a) et b).

**5.** Composition selon l'une des revendications 1 à 4, dans laquelle pour chaque double liaison dans le composant a) 1,5 à 4 molécules de dérivé de styrène sont présents dans la composition.

**6.** Utilisation de la composition selon l'une quelconque des revendications 1 à 5, dans l'une des méthodes de traitement suivantes: le revêtement, la peinture, le moulage par coulée, l'immersion, le laminage, l'imprégnation de crevasses, le jetage, le collage, l'injection de résine, le moulage par compression, le moulage par injection, l'étirement de profile, le masticage, et l'enroulage.

**7.** Procédé pour la production de produits durcis, dans lequel une composition selon l'une quelconque des revendications 1 à 5 durcie.

**8.** Produit avec une activité antimicrobienne contenant une composition durcie selon la revendication 7.

**9.** Utilisation du produit selon la revendication 8 pour la fabrication des produits suivants: les meubles et les surfaces de meubles, les adhésifs, les placages et les papiers stratifiés, les boutons, les poignées, les touches, les interrupteurs et les boitiers, les planches, les planchers, les tuyaux, les profilés, les citernes et les conteneurs, en particulier pour l'eau potable, la nourriture et les huiles, les revêtements, les revêtements de toit, les panneaux lumineux, les mastics, les masses a cheviller, le béton polymère, le l'aglomarble, les éviers, les receveurs de douche, les baignoires, les lavabos, les sièges de toilette, les meubles de jardin, les clôtures de jardin, les panneaux de façade, les puits de fenêtre de sous-sol, les pièces de véhicules, les support de luminaires, les éoliennes, les imprégnations, les liants, le scellements, les mastics et/ou les mortiers de réaction, les revêtements, les vernis, les revêtements gel, les revêtements supérieurs, les navires, les bateaux, les articles de loisirs.

**10.** Procédé pour la production d'un dérivé de styrène à fonctionnalité amino, dans lequel

a) dans une première étape, une solution aqueuse d'hydroxyde de métal alcalin ayant une concentration comprise dans une plage de 3 à 7 moles / l est pourvue,

b) dans une deuxième étape, à cette solution aqueuse d'hydroxyde de métal alcalin, une quantité demi-équivalente en mole à équivalente en mole d'une amine ayant au moins un atome d'hydrogène lié à l'atome d'azote est ajoutée,

c) dans une troisième étape, un halogénoalkyl-styrène en une quantité de 0,2 à 0,5 équivalents molaires en fonction de la quantité d'hydroxyde de métal alcalin est ajoutée,

d) dans une quatrième étape, la solution réactionnelle résultante après addition complète de l'halogénoalkylstyrène est ensuite agité durant une période dans une plage de 2 à 48 agitation, et

e) dans une cinquième étape, le dérivé de styrène à fonctionnalité amino résultant est séparé de la solution de réaction restante.

**11.** Utilisation d'un ou d'un mélange de ces trois substances choisies parmi

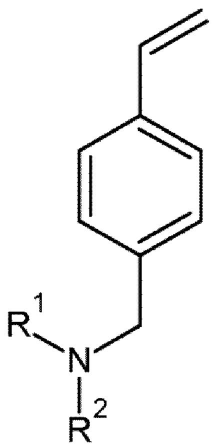

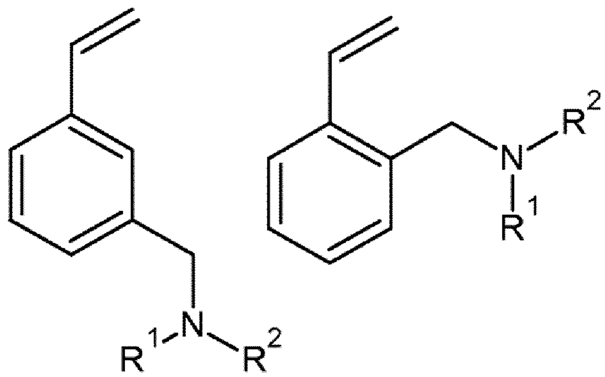

en tant que diluants réactifs dans les applications de résines,
dans laquelle $R^1$ et / ou $R^2$ est indépendamment sélectionné parmi le groupe méthyle, éthyle, n-propyle, iso-propyle, n-butyle, sec-butyle, iso-butyle, tert-butyle, n-pentyle , 3-pentyle, iso-pentyle, tert-pentyle, cyclopentyle, cyclohexyle, dans laquelle $R^1$ peut également être H.

**12.** Matériau choisi parmi

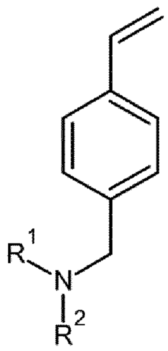

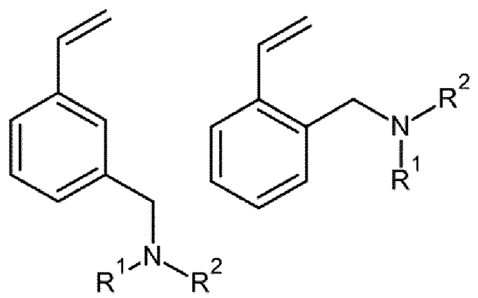

dans lequel $R^1$ est un atome d'hydrogène et $R^2$ représente du tert-pentyle.

**13.** L'utilisation du dérivé de styrène à fonctionnalité amino selon la revendication 11 ou 12 ou obtenu selon la revendication 10 pour la préparation de revêtements ou de corps moulés antimicrobiens.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6242526 B
- US 4810567 A
- US 5614568 A
- US 4447580 A
- JP 2000239281 A
- US 6200680 B
- US 4021416 A
- DE 10242561 A1
- DE 4432985 A1
- DE 19723504 C1


### In der Beschreibung aufgeführte Nicht-Patentliteratur


- **KUNO et al.** *Reactive & Functional Polymers,* 2000, vol. 43, 43-51
- **H. MENZEL.** Schutzschicht gegen Bakterien. *Nachrichten aus der Chemie,* November 2011, vol. 59, 1039-43
- Faserverstärkte Kunststoffe und duroplastische Formassen. AVK-TV-Handbuch. 2005, 40